# EUROPEAN PATENT SPECIFICATION

(11) **EP 4 604 830 B1**
(45) Date of publication and mention of the grant of the patent: **17.06.2026**
(21) Application number: 23790281.2
(22) Date of filing: 13.10.2023
(51) Int. Cl.: A61B 5/022, A61B 5/00, A61B 5/0225

(54) **ARTEFACT DETECTION METHOD FOR HEMODYNAMIC PARAMETER MEASUREMENT**
ARTEFAKTDETEKTIONSVERFAHREN ZUR HÄMODYNAMISCHEN PARAMETERMESSUNG
PROCÉDÉ DE DÉTECTION D'ARTEFACTS POUR LA MESURE DE PARAMÈTRES HÉMODYNAMIQUES

(30) Priority: 18.10.2022 EP 22202103
(43) Date of publication of application: 27.08.2025
(73) Proprietor: Koninklijke Philips N.V., 5656 AE Eindhoven (NL)
(72) Inventor: WIJSHOFF, Ralph Wilhelm Christianus Gemma Rosa, 5656 AG Eindhoven (NL); MENA BENITO, Maria Estrella, 5656 AG Eindhoven (NL); DERKX, Rene Martinus Maria, 5656 AG Eindhoven (NL); DAVIDOIU, Valentina-Geta, 5656 AG Eindhoven (NL)
(74) Representative: Philips Intellectual Property & Standards
(86) International application number: PCT/EP2023/078529
(87) International publication number: WO 2024/083684

(56) References cited:
- US-A1- 2009 156 946
- US-A1- 2021 121 073

## Description

### FIELD OF THE INVENTION

The present invention relates to artefact detection in the field of measurement of blood pressure and other hemodynamic parameters.

### BACKGROUND OF THE INVENTION

US2021121073A1 relates to an apparatus and a method of operating the apparatus for use with a wearable cuff in determining blood pressure and/or pulse rate.

Physiological signals provide an important input to clinical decision-making by health care professionals. However, the accuracy of the information collected depends on the quality of the recorded data.

An unreliable signal or a poor signal quality may lead to incorrect measurements, false alarms and/or inappropriate clinical decisions.

One source of measurement error is signal artefacts. Some artefacts may be highly transient, while others may be long lasting.

Artefacts within the context of cuff-based blood pressure measurement (and/or other hemodynamic parameters) may, by way of non-limiting example, have different causes such as: patient motion; cuff movement during measurement; and measurement technique artefacts due to cuff cracks, where a sudden loosening of the cuff securement leads to a sudden increase in cuff volume, and a consequent reduction in measured pressure signals.

In some cases, artefacts may go unnoticed during the procedure by medical care staff, which can result in inaccurate measurements, erroneous data and potentially inappropriate clinical assessment. Ina ppropriate treatment (or inappropriate lack of treatment) may follow.

In some other cases, the artefacts may only be noticed after the measurement has finished. In these cases, the solution depends on each specific situation. For example, it may require repeating the measurement, which adds an additional workflow step and additional time. Moreover, if the actual cause of the artefact is not known, the repeated measurement may result in the same artefact recurring.

Furthermore, by repeating a measurement, the comfort of the patient is compromised because the hemodynamic measurement may take on average 90 seconds and can be uncomfortable for the patient.

There is a need for a means for detecting artefacts and preferably for taking or recommending actions to remedy them.

### SUMMARY OF THE INVENTION

The invention is defined by the independent claims. The dependent claims define advantageous embodiments.

According to examples in accordance with an aspect of the invention, there is provided a method for use during measurement of blood pressure and/or other hemodynamic parameters by a hemodynamic parameter measurement apparatus. The hemodynamic parameter measurement apparatus with which the method might be used may comprise a cuff for wrapping around a part of the body (e.g. arm) of a user, and wherein the cuff includes a pneumatic actuator in the form of an inflatable bladder for use in changing a pressure applied to the body part by the cuff, and may further include a tissue pressure sensor arranged for sensing a pressure between a surface of the user's body part and the cuff, and an operational parameter sensing means for sensing an operational parameter of the pneumatic actuator. For example, the operational parameter sensor may include an actuation pressure sensor for sensing a pressure within the inflatable bladder. For example, the cuff may be configured such that at least a pressure-sensitive part of the tissue pressure sensor is arranged in use between the body part and the cuff.

The method itself comprises receiving from the hemodynamic parameter measurement apparatus a set of sensor signals, wherein the set of sensor signals includes: a tissue pressure signal indicative of said pressure between a surface of the user's body part and the cuff; and an actuator operational signal indicative of an operational parameter of the pneumatic actuator. The method further comprises processing both signals with an artefact detection module to detect, based on a combination of the signals, the occurrence of any of a pre-defined set of artefact events of different types, each artefact event type corresponding to a physical event affecting sensor readings. The method may further comprise generating an output based on the detected artefacts event(s). For example, the method may comprise generating a report indicative of the type of any one or more detected artefact events, and preferably exporting the report as a data item.

Thus, embodiments of the present invention are based on the idea of detecting physical events, such as physical disturbances, associated with the measurement apparatus or the patient which may lead to signal artefacts based on analysis applied to the tissue pressure signal and at least one operational parameter signal of the pneumatic actuator. The use of both of these signals in detecting artefacts has not before been proposed. Through experimentation and simulation it has been found by the inventors that certain physical disturbance events associated with the apparatus or the patient lead to detectable features within the two previously mentioned signal waveforms, and thus provide a way for detecting artefacts and potentially taking action to improve measurements or at least annotate measurements with warning markers where artefacts are potentially present.

The measurement apparatus may be for use in measuring blood pressure, and/or one or more other hemodynamic parameters such as cardiac output and/or stroke volume.

The aforementioned tissue pressure sensor for example may comprise a sensor pad arranged to be applied by the cuff against the body part when the cuff is worn. The sensor pad may be a fluid-filled sensor pad. The fluid pad may be fluidly connected with a pressure transducer of the tissue pressure sensor, wherein the pressure transducer is adapted to convert changes in fluid pressure within the fluid pad to an electronic pressure readout. The pressure transducer might be located outside of the cuff, and the pad may be arranged as a part of the cuff.

The aforementioned physical event to which an artefact event corresponds may for example be a physical disturbance of the apparatus (such as the cuff or tubing pneumatically supplying the cuff or a fluid tube connecting a fluid-filled sensor pad comprised by the tissue pressure sensor to a pressure transducer), or of the patient (e.g. movement of the patient).

In some embodiments, the method is performed in real time during a hemodynamic parameter measurement such as a blood pressure measurement. This allows for remedial action to be taken to correct for artefacts (such as repeating measurements) while the patient is still present.

In some embodiments, the artefact detection is performed based on detecting one or more characteristic features in a waveform of one or more of the received sensor signals. This can be done using classical signal processing techniques. Additionally or alternatively, it is also possible to use machine learning algorithms to detect occurrence of one or more artefact events based on inputs comprising the set of sensor signals. This can be achieved through suitable training of the algorithms with training data comprising sample sensor signal waveforms annotated according to whether the signal waveforms are associated with a particular one or more artefacts.

In some embodiments, the artefact detection includes detection of a motion artefact based on: high pass filtering the actuator operational signal, and detecting a power in the high-pass filtered signal which exceeds a predefined threshold. By 'power' may be meant for example a strength of an AC component of the high-pass-filtered signal. This could e.g. be computed as the mean of the squared high-pass filtered signal samples over a time window. Alternatively, it could be computed as the mean of the absolute values of the high-pass filtered samples over a time window. In either case, the resulting metric is indicative of a strength of the AC component of the signal, i.e. a power of the signal.

In some embodiments, the artefact detection includes detection of a motion artefact based on computing a self-correlation measure of the tissue pressure signal, and detecting that the self-correlation measure falls below a threshold.

A self-correlation measure is indicative of how well the tissue pressure signal is correlated with itself over time.

In some embodiments, the detection includes detection of a motion artefact based on: a first detection comprising high pass filtering the actuator operational signal, and detecting a power in the high-pass filtered signal at any time point which exceeds a predefined threshold; and a second detection comprising computing a self-correlation measure of the tissue pressure signal, and detecting that the self-correlation measure at any time point falls below a threshold. A motion artefact may be detected in the event that the time point to which the first detection corresponds and the time point to which the second detection corresponds are within a pre-defined proximity range of one another. The time points may be the same, or within a threshold time range of one another.

In some embodiments, the set of sensor signals further includes a position and/or motion sensor signal indicative of pose and/or motion of the patient.

The method may comprise detecting occurrence of an artefact event comprising a change in pose of the patient or a motion of the patient, for example using the position and/or motion sensor signal indicative of pose and/or motion of the patient.

In some embodiments, the tissue pressure sensor comprises a fluid-filled sensor pad arranged such that a pressure between the surface of the user's body part and the cuff is applied onto the fluid-filled pad. The fluid-filled sensor pad may be arranged in use between the actuator of the cuff and the body part of the user. The fluid-filled pad may be fluidically connected to a pressure transducer by a fluid line and wherein a pressure applied to the fluid pad is electronically converted to a pressure reading by the pressure transducer.

In some embodiments, the artefact detection includes detection of an artefact event corresponding to a user touching a fluid line or tube connecting a fluid-filled sensor pad comprised by the tissue pressure sensor to a pressure transducer. Here, the detection may comprise: computing a self-correlation measure of the tissue pressure signal over a time period; and computing a noise measure of the actuator operational signal, the noise measure indicative of a degree of variation of the signal from a smooth ramp function over the time period. The touching of the fluid line may be detected responsive to the self-correlation of the tissue pressure signal being below a pre-defined threshold, and the noise measure of the actuator operational signal being below a pre-defined threshold.

To explain, if the fluid-filled tube of the tissue-pressure measurement system is accidentally touched, this will result in a "ringing pattern" in the tissue pressure signal, while it is expected to not affect the actuator operational parameter signal. The "ringing pattern" can be recognized in the tissue pressure signal from a reduction in a correlation signal which shows how well the tissue pressure signal is correlated with itself over time.

Alternatively, as the "ringing pattern" depends on the tube length and fluid type, one can define a template or signature for the expected "ringing pattern" (e.g., resonant frequency and damping coefficient) which one can use to identify such a "ringing pattern" in the tissue pressure signal. In case such a "ringing pattern" is detected in the tissue pressure signal, while the actuator operational parameter signal remains unaffected, one obtains an indication that the fluid-filled tube has been touched.

Absence of artefacts in the actuator operational signal can be detected by assessing whether the actuator operational signal takes the form of a ramp signal (i.e. smoothly escalating pressure) which increases over time without any (oscillatory) signal components or inflection.

Thus, in some embodiments, the artefact detection may include detection of an artefact event corresponding to a user touching a fluid line connecting a fluid-filled sensor pad comprised by the tissue pressure sensor to a pressure transducer comprised by the tissue pressure sensor, and wherein the detection comprises detecting one or more pre-defined characteristic waveform patterns within the waveform of the tissue pressure signal.

In some examples the detection may also depend on detecting absence of said waveform pattern in the actuator operational signal and/or detecting absence of any oscillatory components or inflections in the actuator operational signal (or the baseline of the operational signal).

In some embodiments, the cuff includes a plurality of at least partially overlapping layers or layer portions, which for example are slidable relative to one another to vary a diameter of the cuff. In embodiments, the artefact detection includes detection of an artefact event corresponding to stick-slip event in relation to said overlapping layers. For example, in some embodiments, the cuff may include a shell structure, wherein the shell structure is arranged so as to be located between the pneumatic actuator and the body part when the cuff is worn on the body part, and arranged to surround the body part when the cuff is worn. The shell structure may include at least partially overlapping portions, and wherein the at least partially overlapping portions of the shell structure may move or slide relatively to each other, thereby reducing the diameter of the shell structure responsive to increasing applied pressure by the pneumatic actuator. The artefact detection may include detection of an artefact event corresponding to stick-slip event in relation to said at least partially overlapping portions.

In some embodiments, the shell structure may comprise a single sheet or layer element which is rolled into a tubular shape, and wherein circumferential ends of the rolled sheet or layer overlap to form the at least partially overlapping portion(s).

The stick-slip event may be detected based on: detecting an upward inflection in the tissue pressure signal exceeding a first pre-defined gradient threshold, and detecting a downward inflection in the actuator operational signal exceeding a second pre-defined gradient threshold. The inflections may, in particular, be inflections in the baselines of the respective signals.

During a stick-slip artefact, there is a friction within the shell that blocks a gradual decrease of the circumference of the shell over time. At regular moments, this friction is 'released/removed' and the following chain of reactions occurs: sudden decrease of the shell circumference; sudden decrease of the actuator operational parameter, e.g. actuator pressure; sudden increase of the tissue pressure.

In some embodiments, the artefact detection includes detection of an artefact event corresponding to a slippage or loosening of a cuff securement means. The detection here may be based on: detecting a downward inflection in the actuator operational signal exceeding a first pre-defined gradient threshold, and detection of a downward inflection in the tissue pressure signal exceeding a second pre-defined gradient threshold. The inflections may, in particular, be inflections in the baselines of the respective signals.

In particular, at some moments, the Velcro of the cuff air bladder can loosen as a result of which the shell gets a (slightly) larger circumference. This is known in the art as cuff 'cracking'. Due to the larger circumference, it is expected that one would observe: a sudden decrease of the tissue pressure signal; and a sudden decrease of the actuator operational parameter, such as actuator pressure.

In some embodiments, the detection further comprises detecting the downward inflection in each of the tissue pressure and actuator operational signals followed by a signal portion of each respective signal representing a negative offset relative to the respective signal prior to the downward inflection; and wherein the detection further comprises determining whether a time duration of said signal portion exceeds a pre-defined threshold.

After the artefact, both tissue pressure and actuator operational parameter (e.g. actuator pressure) show an additional sustained (negative) offset.

In some embodiments, the actuator operational parameter sensing means comprises an actuation pressure sensor for sensing a pressure within the inflatable bladder. In some embodiments, the actuator operational signal comprises an actuator pressure signal indicative of a pressure within the inflatable bladder.

In some embodiments, the actuator operational signal includes an actuator activity signal indicative of a pumping power level or pumping rate of a pump of the pneumatic actuator.

As a more general statement, the actuator operational signal may be a signal directly or indicative of an actuation state of the actuator, for example indicative of a physical position or inflation state of the actuator.

In some embodiments, responsive to detection of an artefact, the method comprises generating a control signal for output to the hemodynamic parameter measurement apparatus for controlling the apparatus to abort a current hemodynamic parameter measurement.

In some embodiments, the method further comprises determining a recommended response action to be taken by a user based on application of a recommendation module to the output of the artefact detection module. In some embodiments, the generated report is further indicative of the recommended response action for each detected artefact.

In some embodiments, the recommended response action is to repeat the hemodynamic parameter measurement. Additionally or alternatively, in some embodiments, the recommended response action is to re-fit (e.g. re-position or re-wrap) the cuff of the hemodynamic parameter measurement apparatus to the user and repeat the hemodynamic parameter measurement. Additionally or alternatively, in some embodiments, the recommended response action is to replace the cuff of the hemodynamic parameter measurement apparatus with a new cuff and repeat the hemodynamic parameter measurement.

In some embodiments, the report is generated and exported at an end of a complete measurement cycle of the hemodynamic parameter measurement apparatus, or is generated and exported during the hemodynamic parameter measurement cycle, immediately upon detection of a given artefact event.

Another aspect of the invention is a computer program product comprising code means configured for being run on a processor operatively coupled to a hemodynamic parameter measurement apparatus, the hemodynamic parameter measurement apparatus comprising a cuff for wrapping around a part of the body of a user, and wherein the cuff includes a pneumatic actuator in the form of an inflatable bladder for use in changing a pressure applied to the body part by the cuff, and further includes a tissue pressure sensor arranged for sensing a pressure between a surface of the user's body part and the cuff, and an operational parameter sensing means for sensing an operational parameter of the pneumatic actuator. The code means is configured, when run, to cause the processor to perform a method in accordance with any embodiments or example described in this document, or in accordance with any claim of this application. The operational parameter sensing means may in some embodiments be an actuation pressure sensor for sensing a pressure within the inflatable bladder. In some embodiments, the actuator operational signal includes an actuator activity signal indicative of a pumping power level or pumping rate of a pump of the pneumatic actuator.

Another aspect of the invention is a processing unit adapted for a hemodynamic parameter measurement apparatus, comprising: an input/output adapted to be operatively coupled in use with a hemodynamic parameter measurement apparatus, the hemodynamic parameter measurement apparatus comprising a cuff for wrapping around a part of the body of a user, and wherein the cuff includes a pneumatic actuator in the form of an inflatable bladder for use in changing a pressure applied to the body part by the cuff, and further includes a tissue pressure sensor arranged for sensing a pressure between a surface of the user's body part and the cuff, and further includes an operational parameter sensing means for sensing an operational parameter of the pneumatic actuator; and one or more processors configured to execute a method. The operational parameter sensing means may in some embodiments be an actuation pressure sensor for sensing a pressure within the inflatable bladder. In some embodiments, the actuator operational signal includes an actuator activity signal indicative of a pumping power level or pumping rate of a pump of the pneumatic actuator. The cuff may be configured such that at least a pressure-sensitive part of the tissue pressure sensor is arranged between the body part and the actuator when the cuff is worn by the user. The tissue pressure sensor may comprise a fluid filled sensing pad fluidly connected to a pressure transducer for reading out changes in pressure applied to the sensor pad. The cuff may be configured such that the fluid filled sensor pad is arranged between the actuator of the cuff and the body part when the cuff is worn by the user.

The method comprises: receiving from the hemodynamic parameter measurement apparatus, via the input/output, a set of sensor signals, wherein the set of sensor signals includes: a tissue pressure signal indicative of said a pressure between a surface of the user's body part and the cuff, and an actuator operational signal indicative of an operational parameter of the pneumatic actuator. The method further comprises processing both signals with an artefact detection module to detect, based on a combination of the signals, the occurrence of any of a pre-defined set of artefact events of different types, each artefact event type corresponding to a physical event affecting sensor readings. The method may further comprise generating an output based on the detected occurrence of any artefact event(s). The method may for example comprise generating a report indicative of the type of any one or more detected artefact events, and exporting the report as a data item via the input/output.

Another aspect of the invention is a system. The system comprises a processing unit in accordance with any embodiment described in this disclosure, for example in accordance with the processing unit outlined above. The system further comprises a hemodynamic parameter measurement apparatus, operatively coupled with the processing unit, the hemodynamic parameter measurement apparatus comprising a cuff for wrapping around a part of the body of a user, and wherein the cuff includes a pneumatic actuator in the form of an inflatable bladder for use in changing a pressure applied to the body part by the cuff, and further includes a tissue pressure sensor arranged for sensing a pressure between a surface of the user's body part and the cuff, and an actuation operational parameter sensing means for sensing an operational parameter of the pneumatic actuator, for example an actuator pressure sensor for sensing a pressure within the inflatable bladder or an actuator activity signal indicative of a pumping power level or pumping rate of a pump of the pneumatic actuator. The cuff may be configured such that at least a pressure-sensitive part of the tissue pressure sensor is arranged between the body part and the actuator when the cuff is worn by the user. The tissue pressure sensor may comprise a fluid filled sensing pad fluidly connected to a pressure transducer for reading out changes in pressure applied to the sensor pad. The cuff may be configured such that the fluid filled sensor pad is arranged between the actuator of the cuff and the body part when the cuff is worn by the user. These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically illustrates an example hemodynamic parameter measurement cuff suitable for use in methods according to embodiments of the present invention;
Fig. 2 outlines steps of an example method in accordance with one or more embodiments of the invention;
Fig. 3 shows a block diagram of components of an example processing unit and system in accordance with one or more embodiments of the invention;
Fig. 4 shows a processing flow according to one or more embodiments of the invention;
Fig. 5 illustrates an example recording of a tissue pressure signal and actuator pressure signal obtained during a measurement of hemodynamic parameters using a hemodynamic parameter measurement apparatus;
Fig. 6 illustrates an illustrative characteristic waveform feature permitting detection of a stick-slip artefact; and
Fig. 7 illustrates a characteristic waveform feature permitting detection of a cuff crack artefact.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs. are merely schematic and are not drawn to scale. It should also be understood that the same reference numerals are used throughout the Figs. to indicate the same or similar parts.

The invention provides a means for detecting and classifying artefact-causing events associated with a hemodynamic parameter measurement based on analysis of a tissue pressure signal indicative of a pressure between a surface of the user's body part and a cuff of the measurement apparatus; and an actuator operational signal indicative of an operational parameter of a pneumatic actuator associated with the cuff.

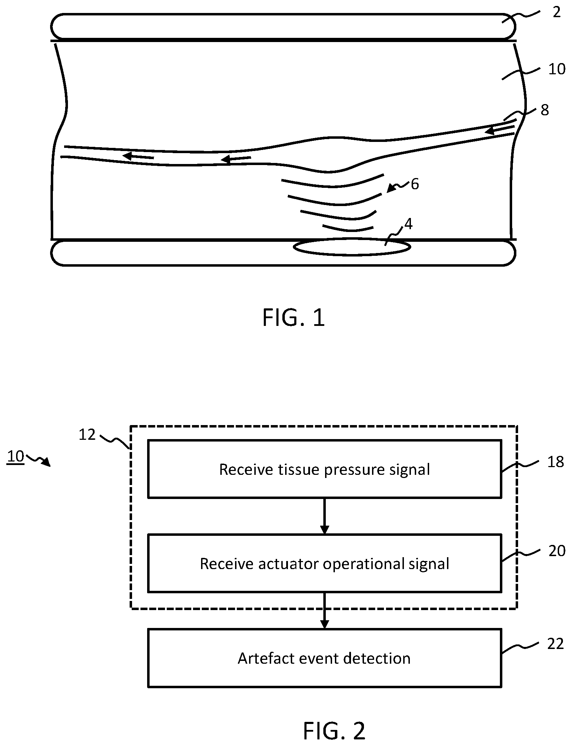

By way of background, Fig. 1 shows a schematic illustration of an example cuff-based hemodynamic parameter measurement apparatus with which embodiments of the present artefact-detection method may advantageously be applied. The apparatus can be used to detect blood pressure and/or other hemodynamic parameter measurements such as cardiac output or stroke volume. The illustration shows the cuff 2 applied to the upper arm 10 of a patient. An artery 8 of the patient is schematically shown. The apparatus has a design which differs from the most standard cuff-based blood pressure measurement apparatuses in that it includes a dedicated tissue pressure sensor 4 which the cuff acts to hold against the tissue of the skin when the cuff is inflated. The cuff 2 includes a pneumatic actuator in the form of an inflatable bladder for use in changing a pressure applied to the body part 10 by the cuff. The tissue pressure sensor 4 is arranged for sensing a pressure between a surface of the user's body part and the cuff. Independently of the tissue pressure sensor, one or more operational parameters of the pneumatic actuator can be sampled. For example, a pressure inside the inflatable bladder can be sensed. An actuator activity signal indicative of a pumping power level or pumping rate of a pump of the pneumatic actuator can also be sampled.

Standard blood pressure measurement cuffs use the bladder air pressure as the sole measurement signal for sensing blood pressure. By additionally utilizing a tissue pressure sensor 4, the apparatus shown in Fig. 1 can achieve superior quality results and also enables the measurement of advanced hemodynamic parameters such as stroke volume, cardiac output and fluid responsiveness in addition to blood pressure. In particular, in standard air blood pressure cuffs, dampening air is assumed to destroy > 90% of the amplitude and contour of a tissue pressure pulse wave. By contrast, by using a dedicated tissue pressure sensor, the design illustrated in Fig. 1 allows recording high-fidelity (HiFi) artery blood pressure and pulse waveform.

The tissue pressure sensor 4 may be implemented as, e.g. a fluid-filled bag which provides a conforming layer of fluid between the cuff 2 and the surface of the user's body. In this way, the integrated pneumatic actuator enables hydraulic coupling of the tissue pressure sensor 4 to the rigid shell-encased upper arm tissue. When blood pulses in the artery, this results in a pressure wave 6 which can be detected by the tissue pressure sensor 4. The tissue pressure sensor 4 can be connected to a pressure transducer via a fluid-filled tube/line. The pressure transducer converts the pressure in the fluid into an electrical signal.

The operating principle is based on coupling of the pressure sensor to the body part (e.g. arm) in order to transcutaneously record tissue pressure pulse waves that result from arterial pulsation (e.g. brachial artery). In similar fashion to a conventional upper arm air blood pressure cuff, the cuff compresses the upper arm using an integrated pneumatic actuator with rising clamping pressure. However, the actual compression may be achieved via the narrowing diameter of a rigid circumferential shell.

In some embodiments, the cuff 2 may include a shell structure, wherein the shell structure is arranged so as to be located between the pneumatic actuator and the body part when the cuff is worn on the body part, and arranged to surround the body part when the cuff is worn. The shell structure may be relatively stiff. Consequently, the measurement accuracy can be improved, since the pressure in the arm is measured against a relatively stiff support, which prevents dampening of the amplitude and shape of the signal. In particular, if a tissue pressure sensor unit is located at least partially between the shell structure and the body part, a high accuracy of the signals measured by the tissue pressure sensor unit can be achieved. With such a configuration of the tissue pressure sensor unit, the shell structure does not absorb or attenuate the arterial pressure signal.

The shell structure may include at least partially overlapping portions, and wherein the overlapping portions of the shell structure may move or slide relatively to each other, thereby reducing the diameter of the shell structure responsive to increasing applied pressure by the pneumatic actuator.

In some embodiments, the shell structure may comprise a single sheet or layer element which is rolled into a tubular shape, and wherein circumferential ends of the rolled sheet or layer overlap to form the at least partially overlapping portion(s).

The design described above permits non-invasive hemodynamic monitoring and enables measurement of blood pressure, as well as cardiac output and other hemodynamic parameters.

For more expansive details about this example hemodynamic parameter measurement apparatus, reference is made to the document EP2953528 A1 which describes the cuff design in more detail. The hemodynamic parameter measuring apparatus according to embodiments of the present invention may be implemented in accordance with the hemodynamic parameter measuring system described in the document EP2953528 A1.

As has been discussed above, artefacts can occur in the signals output by the hemodynamic parameter measurement apparatus. This can lead to inaccuracy in any measurements obtained based on processing of these signal, for example different hemodynamic measurements, as well as the blood pressure calculation itself.

Different artefacts have different causes and require different responses to remedy them. Embodiments of the present invention are based on detecting signal waveform features which are indicative of certain physical disturbance events of the hemodynamic parameter measurement apparatus or of the patient. By detecting these artefact-causing events, the timings of likely signal artefacts are detected.

Some artefacts require repeating the measurement. Here, knowing the cause of the artefact is helpful since the repetition of the measurement can be done in such a way as to prevent the artefact-causing event from occurring again.

For some artefacts, when they occur too frequently, the user may consider stopping the measurement and re-applying or replacing the cuff. In this case, it is helpful if the system can recognize this situation and provide the appropriate recommendation to re-apply or replace the cuff.

In some other cases, a correction can be performed to negate the artefacts.

One aim of embodiments of the present invention is to at least partially overcome the shortcomings of the state of the art devices by providing an approach to detect, identify and communicate the type of artefacts to the user plus preferably a recommended response action, so that the clinician can determine what remedial action needs to be taken or if e.g. a correction of the measurement needs to be applied, or if the artefact needs to be taken into account in the assessment of the measurement results.

In various embodiments of the invention, we propose an approach to detecting and managing artefacts which can occur in state of the art hemodynamic parameter measurement devices during measurement of blood pressure (BP), stroke volume (SV), cardiac output (CO), fluid responsiveness (FRP) and other hemodynamic (HDM) parameters.

Fig. 2 outlines in block diagram form steps of an example method according to one or more embodiments. The steps will be recited in summary, before being explained further in the form of example embodiments.

The method is a computer implemented method for use during hemodynamic parameter measurement by a hemodynamic parameter measurement apparatus, wherein the hemodynamic parameter measurement apparatus comprises a cuff for wrapping around a part of the body of a user, and wherein the cuff includes a pneumatic actuator in the form of an inflatable bladder for use in changing a pressure applied to the body part by the cuff, and further includes a tissue pressure sensor arranged for sensing a pressure between a surface of the user's body part and the cuff, and an operational parameter sensing means for sensing an operational parameter of the pneumatic actuator.

The method comprises receiving 12 from the hemodynamic parameter measurement apparatus a set of sensor signals. This includes receiving 18 a tissue pressure signal indicative of said pressure between a surface of the user's body part and the cuff. It further includes receiving 20 an actuator operational signal indicative of an operational parameter of the pneumatic actuator. The method further comprises processing 22 both signals with an artefact detection module to detect, based on a combination of the signals, the occurrence of any of a pre-defined set of artefact events of different types, each artefact event type corresponding to a physical event affecting sensor readings. The method preferably may further comprise generating an output indicative of the detected occurrence of any of the pre-defined set of artefact events of different types. For example, the method may comprise generating a report indicative of the type of any one or more detected artefact events, and preferably exporting the report as a data item

As noted above, the method 10 can also be embodied in hardware form, for example in the form of a processing unit which is configured to carry out a method in accordance with any example or embodiment described in this document, or in accordance with any claim of this application.

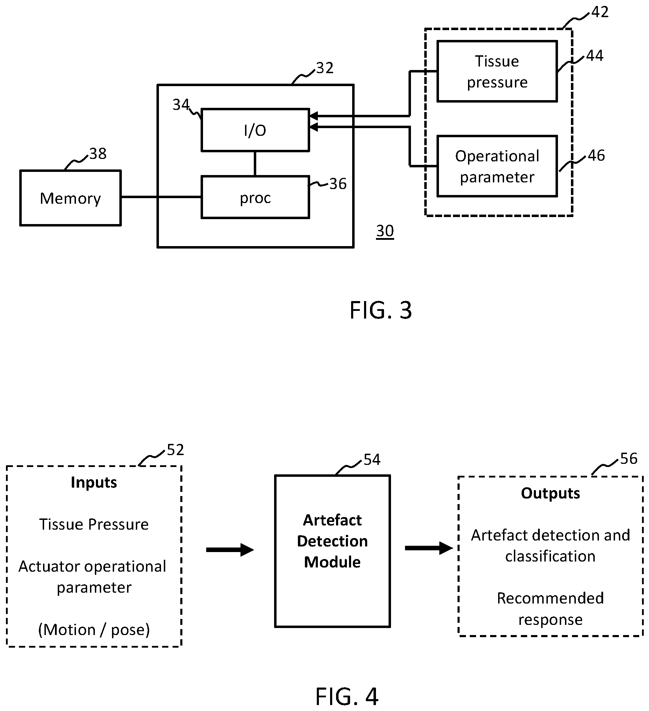

To further aid understanding, Fig. 3 presents a schematic representation of an example processing unit 32 configured to execute a method in accordance with one or more embodiments of the invention. The processing unit is shown in the context of a system 30 which comprises the processing unit. The processing unit 32 alone represents an aspect of the invention. The system 30 is another aspect of the invention. The provided system does not have to comprise all of the illustrated hardware components; it may just comprise a subset of them.

The processing unit 32 comprises one or more processors 36 configured to perform a method in accordance with that outlined above, or in accordance with any embodiment described in this document or any claim of this application. In the illustrated example, the processing unit further comprises an input/output 34 or communication interface.

In the illustrated example of Fig. 3, the system 30 further comprises a hemodynamic parameter measurement apparatus 42 which comprises a tissue pressure sensor 44 arranged for sensing a pressure between a surface of the user's body part and a cuff of the apparatus, and an operational parameter sensing means 46 for sensing an operational parameter of the pneumatic actuator. For example, the hemodynamic parameter measurement apparatus 42 may comprise a cuff for wrapping around a part of the body of a user, and wherein the cuff includes a pneumatic actuator in the form of an inflatable bladder for use in changing a pressure applied to the body part by the cuff. In some embodiments, the operational parameter sensing means may include at least an actuation pressure sensor for sensing a pressure within the inflatable bladder. In some embodiments, the operational parameter sensing means may include an actuator activity signal indicative of a pumping power level or pumping rate of a pump of the pneumatic actuator.

The system 30 may further comprise a memory 38 for storing computer program code (e.g. computer-readable code) which is configured for causing the one or more processors 36 of the processing unit 32 to perform the method as outlined above, or in accordance with any embodiment described in this disclosure, or in accordance with any claim.

Fig. 4 shows a flow diagram which outlines the basic processing flow according to one or more embodiments. The inputs 52 to the processing operation may, as noted above, include a tissue pressure signal from the tissue pressure sensor, actuator operational parameter signal (such as actuation pressure or a pumping power level or pumping rate of a pump), and optionally a motion or position signal for indicating motion or pose of the patient (such as acquired via e.g. an accelerometer or gyroscope coupled to the patient). These inputs are provided to an artefact detection module 54 which analyzes one or more of the signals to detect the artefact features in the signals and classify the type of artefact causing event leading to the artefact features present in one or more of the input signals. The output 56 is the detected type of artefact event plus optionally a recommendation to the user of a best follow-up action to remedy or mitigate the artefact. By way of example, the recommended follow-up action could be at least any or a combination of the following actions: repeat the measurement; reapply or replace the cuff and repeat the measurement; retain the measurement but mark the measurement as potentially unreliable due to the artefact; apply a model-based correction of the artefact.

With regards to the artefact detection module 54, this may comprise one or more algorithms configured to process the received set of sensor signals and generate the output detection of the one or more artefact events of different types. Each of the one or more algorithms may be adapted to perform signal processing to detect one or more characteristic features in a waveform of one or more of the received sensor signals. The module may be operable to execute a plurality of detection algorithms, each algorithm specialized to detecting a particular type of artefact event. By applying the plurality of algorithms, the set of signals is checked for presence of each of the possible types of artefacts. Additionally or alternatively, in some embodiments, the artefact detection module may be operable to execute a plurality of detection modules, each configured to detect a respective one or more characteristic features in a waveform of one or more of the received sensor signals. The outputs of these detection modules might then be used to detect the presence of one or more particular types of artefact event, where each particular type of artefact event is characterized by presence of a particular combination of the different possible characteristic waveform features.

By way of further example, the artefact detection module may employ use of one or more machine learning algorithms which have been pre-trained to detect one or more types of artefact event based on the input signals. Again, there may be a plurality of machine learning algorithms in some cases, each trained to detect a particular type of artefact event based on a particular combination of signals.

There will now be described in more detail how the input set of sensor signals may be processed to detect particular types of artefacts.

In particular, the artefact detection module 54 may be configured to automatically detect and classify one or more of the following artefacts:
a motion artefact, corresponding to motion of the patient or change in pose or posture of the patient;
an artefact event corresponding to a user touching a fluid line of the hemodynamic parameter measurement apparatus which connects a fluid-filled sensor pad of a pressure sensing means to a pressure transducer;
a stick-slip event in relation to overlapping portions or layers of the hemodynamic parameter measurement cuff (this will be explained in more detail to follow);
a 'cuff crack' event, corresponding to loosening or slippage of a cuff securement means.

Means for detecting patient motion artefact events will first be described.

Motion can be recognized from detecting particular pattern features in the waveform of the tissue pressure signal or the actuator operational parameter signal. As explained above, the tissue pressure signal is indicative of a pressure between a surface of the user's body part and the cuff, and measured by a tissue pressure sensor included in the hemodynamic parameter measurement apparatus. As explained above, the actuator operational signal is indicative of an operational parameter of the pneumatic actuator.

For purposes of the present example, an operational signal in the form of an actuator pressure signal will be used, indicative of a pressure within the inflatable bladder of the pneumatic actuator. By way of further example however, an operational signal in the form of an actuator activity signal indicative of a pumping power level or pumping rate of a pump of the pneumatic actuator may be used.

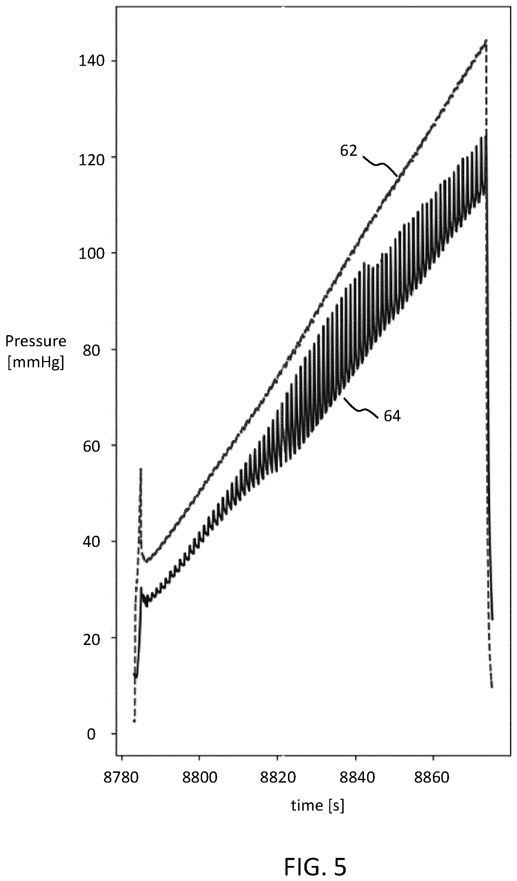

Motion can be detected by detecting an irregular pattern in the tissue pressure or actuator pressure signals. As illustrated in Fig. 5, both the actuator pressure signal (AP) 62 and tissue pressure signal (TP) 64 comprise a baseline ramp function of increasing pressure during the measurement (due to the cuff inflation). Superposed on top of this, the TP signal has high frequency oscillations corresponding to cardiac pulses.

Irregularities in the AP signal which are indicative of motion can be detected if e.g. the signal power level in the high-pass filtered AP signal exceeds a predefined threshold. In other words, the artefact detection may comprise detection of a motion artefact based on: high pass filtering the actuator operational signal, and detecting a signal power level in the high-pass filtered signal which exceeds a predefined threshold. By signal power level is meant a strength of the AC component of the high-pass-filtered signal. This could be an instantaneous power level. Alternatively it could e.g. be computed as the mean of the squared high-pass filtered samples over a time window. Alternatively, it could be computed as the mean of the absolute values of the high-pass filtered samples over a time window. In either case, the resulting metric is indicative of a strength of the AC component of the signal over the relevant window.

With regards to the threshold, this can of course be set in advance based on the particular specifications of the system. For example, this can be programmed during manufacture based on empirical testing.

Irregularities in the TP signal which are indicative of motion can additionally or alternatively be detected from a reduction in a correlation signal which shows how well the TP signal is correlated with itself over time. In other words, the artefact detection may comprise detection of a motion artefact based on computing a self-correlation measure of the tissue pressure signal, and detecting that the self-correlation measure falls below a pre-defined threshold.

A motion event is expected to occur in both the AP and TP signals at a similar moment in time. Therefore corroboration of a motion artefact can be achieved by checking that motion detections occur at similar moments in both the AP and TP signals. In this way, one can detect presence of a motion artefact in the AP and TP signals.

In other words, the detection may include detection of a motion artefact based on: a first detection comprising high pass filtering the actuator operational signal, and detecting a signal power level in the high-pass filtered signal at any time point which exceeds a predefined threshold; and a second detection comprising computing a self-correlation measure of the tissue pressure signal, and detecting that the self-correlation measure at any time point falls below a pre-defined threshold; and wherein a motion artefact is detected in the event that the time point to which the first detection corresponds and the time point to which the second detection corresponds are within a pre-defined proximity range of one another. The time points may be the same, or within a threshold time range of one another.

Furthermore, in some embodiments, a position and/or motion sensor may be included as part of the system 30, arranged to detect a motion of the patient or change in pose or posture of the patient. For example, the position and/or motion sensor may comprise one or more accelerometers, e.g. a tri-axial accelerometer, attached to the patient's body. Using such an accelerometer, changes in the projection of the gravitational component on the three axes of the accelerometer can be detected and this can be used to detect changes in pose or posture of the patient. Such a change is of course indicative of motion of the patient which can cause signal artefacts. A tri-axial accelerometer is not essential. Any accelerometer can be used to detect a motion event associated with the patient. For example, additionally or alternatively, the AC signal power in the motion signal can be detected, and used to detect motion of the patient. For example, if the AC signal power in the motion signal exceeds a pre-defined threshold, this is an indication of motion.

As discussed above, in some embodiments, the tissue pressure sensor can comprise a compressible fluid pad arranged as part of the cuff, and wherein the fluid filled pad is fluidly coupled to a pressure transducer to convert pressures applied physically to the pad (resulting in changes in internal fluid pressure of the pad) to an electronic pressure readout. In this context, another potential event which can physically disturb the hemodynamic parameter measurement, and cause signal artefacts, is if a patient or clinician touches the fluid tube or line which connects the fluid-filled sensor pad to the pressure transducer. If the fluid-filled tube of the tissue-pressure measurement system is accidentally touched, this will result in a "ringing pattern" in the TP signal and is expected to not affect the AP signal.

The "ringing pattern" can be recognized in the TP signal from a reduction in a correlation signal which shows how well the TP signal is correlated with itself over time. Thus, the artefact detection may include detection of an artefact event corresponding to a user touching a fluid line of the hemodynamic parameter measurement apparatus, and wherein the detection comprises: computing a self-correlation measure of the TP signal over a time period; and computing a noise measure of the actuator operational signal, the noise measure indicative of a degree of variation of the signal from a smooth ramp function over the time period; and wherein said touching is detected responsive to the self-correlation of the tissue pressure signal being below a pre-defined threshold, and the noise measure of the actuator operational signal being below a pre-defined threshold.

Additionally or alternatively, the detection may comprise detecting one or more pre-defined characteristic waveform patterns within the waveform of the tissue pressure signal. In particular, as the "ringing pattern" depends on the tube length, the tube diameter and fluid type, it can be characterized according to an expected range for one or more oscillatory properties such as resonant frequency and damping coefficient. Thus, one can define in advance a template or signature waveform pattern for the expected "ringing pattern" (e.g., in terms of expected resonant frequency and damping coefficient) which one can use to identify such a "ringing pattern" in the TP signal.

In case such a "ringing pattern" is detected in the TP signal, one obtains an indication that the fluid-filled tube has been touched. Since the AP is expected at the same time to remain unaffected, in some embodiments, the detection may also depend on detecting absence of said waveform pattern in the actuator operational signal and/or detecting absence of any oscillatory components in the actuator operational signal.

Another class of artefact events are those which represent physical disturbances in the state of the cuff itself.

By way of a first example, the hemodynamic parameter measurement cuff may comprise an arrangement of at least partially overlapping layers or layer portions. In some embodiments, the cuff includes a plurality of overlapping layers or layer portions, which for example are slidable relative to one another to vary a diameter of the cuff. For example, in some embodiments, the cuff may include a shell structure, wherein the shell structure is arranged so as to be located between the pneumatic actuator and the body part when the cuff is worn on the body part, and arranged to surround the body part when the cuff is worn. The shell structure may include overlapping portions, and wherein the overlapping portions of the shell structure may move or slide relatively to each other, thereby reducing the diameter of the shell structure responsive to increasing applied pressure by the pneumatic actuator. In some embodiments, the shell may comprise a single sheet or layer element which is rolled into a tubular shape, and wherein circumferential ends of the rolled sheet or layer overlap to form the overlapping portion.

Stick-slip events can occur between these one or more overlapping layers or portions. During a stick-slip artefact, there is a friction within the shell that blocks a gradual decrease of the circumference of the shell over time. At regular moments, this friction is 'released/removed' and the following chain of reactions occurs: a sudden decrease of the cuff circumference; sudden decrease of the actuator pressure signal (AP), a sudden increase of the tissue pressure signal (TP). The artefact detection may include detection of an artefact event corresponding to stick-slip event in relation to said overlapping portions.

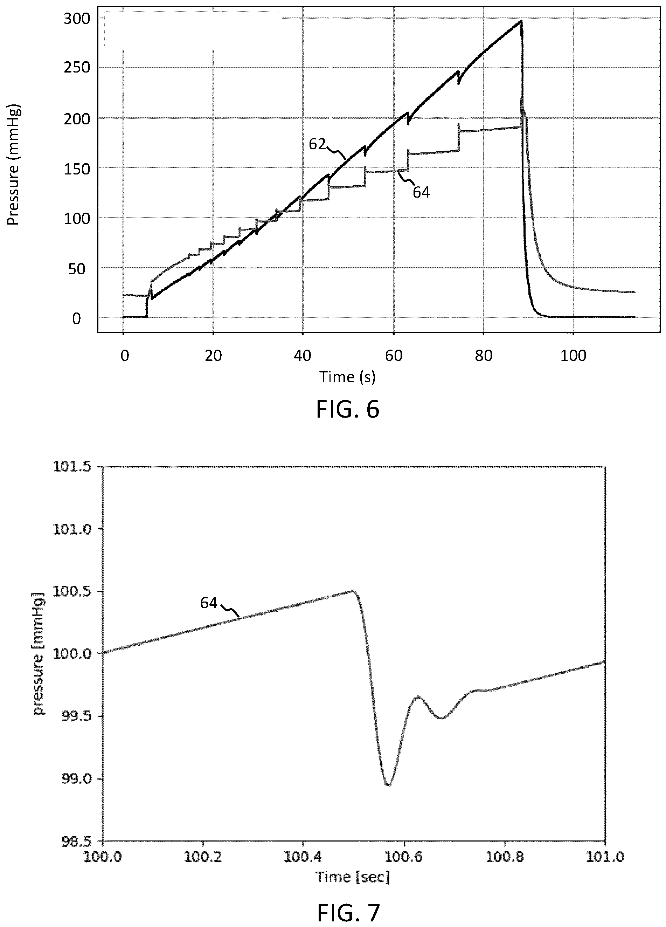

Fig. 6 illustrates examples of the waveform feature in each of the AP 62 and TP 64 signals corresponding to a severe stick-slip event. Fig. 6 in fact shows, for illustration, a sequential series of stick slip events. Each event is characterized in the TP signal 64 by an up-step in the signal, or in other words an upward inflection in the tissue pressure signal exceeding a first pre-defined gradient threshold. The event is characterized in the AP signal 62 by a down-step in the signal, or in other words a downward inflection in the actuator operational signal exceeding a second pre-defined gradient threshold. The inflections or steps may, in particular, be inflections or steps in the baselines of the respective signals. It is noted that the particular example illustrated in Fig. 6 represents a particularly severe artefact event, and thus shows an artefact size somewhat larger than might typically be expected in most cases, and furthermore shows a repeated series of the artefact events. In cases of less severe artefacts, the stick-slip events may present for example with slightly shorter plateaus in the tissue pressure signal 64 and / or slightly smaller steps in the tissue pressure signal 64.

Thus, in cases where the cuff includes a plurality of at least partially overlapping or stacked material layers, detection may be performed of an artefact event corresponding to stick-slip event in relation to said layers, and wherein the stick-slip event is detected based on: detecting an upward inflection in the tissue pressure signal (TP) exceeding a first pre-defined gradient threshold, and detecting a downward inflection in the actuator pressure signal (AP) exceeding a second pre-defined gradient threshold.

Furthermore, it is expected that these inflections would occur at similar moments in time. Thus, the detection could be further corroborated by first detecting occurrence of both of the above-mentioned signal features in the TP and AP signals, and then determining whether they occurred within a pre-defined time window of one another.

The signal features themselves, e.g. the inflections, can for example be detected by using the time-derivative of each of the TP and AP signals.

Another type of artefact which relates to a disturbance of the measurement cuff is the occurrence of so called cuff-cracks. This is a term of art and will be familiar to the skilled person in this field. A cuff crack corresponds to a sudden loosening or slippage of the securement means used to hold the cuff in a wrapped position around the body part. Most usually, this securement means takes the form of hook-and-loop type fastening, e.g. Velcro. At some moments, the Velcro (or other fastening) of the cuff can loosen, as a result of which the cuff gets a slightly larger circumference. Due to the larger circumference, it is expected to observe: a sudden decrease of the tissue pressure and a sudden decrease of actuator pressure. This can be detected by identifying inflections in these signals. The inflections may, in particular, be inflections in the baselines of the respective signals.

In other words, the artefact detection can include detection of an artefact event corresponding to a loosening or slippage of a cuff securement means, and wherein the detection is achieved based on: detecting a downward inflection in the actuator operational signal exceeding a first pre-defined gradient threshold, and detecting a downward inflection in the tissue pressure signal exceeding a second pre-defined gradient threshold.

For illustration, Fig. 7 illustrates the appearance of the signal waveform feature corresponding to a cuff crack in the tissue pressure signal 64. In normal circumstances, during hemodynamic parameter measurement, there is a gradual decrease of the circumference of the cuff as pneumatic actuator pressure increases (the cuff tightens). However, when a cuff crack occurs, both the tissue pressure and actuator pressure inflect downwards suddenly.

In addition, after the artefact, both the tissue pressure and actuator pressure signals show an additional sustained (negative) offset. This is also visible in Fig. 7, where there can be observed a negative "jump" in the TP signal 64 which leads to a sustained negative offset. Therefore, another way to detect the cuff crack is to analyze the TP and AP signals for a sustained negative jump. In other words, the detection may further comprise detecting the downward inflection in each of the tissue pressure and actuator operational signals (as mentioned above) followed by a signal portion of each respective signal representing a negative offset relative to the respective signal prior to the downward inflection; and wherein the detection further comprises determining whether a time duration of said signal portion exceeds a pre-defined threshold.

In accordance with one or more embodiments, the method may include a single processing operation applied to the tissue pressure signal and the actuator operational signal which is configured to detect any of a plurality of different artefacts. This might involve application of a series of detection modules, each configured to detect a particular one of a set of characteristic waveform features, and wherein different of said set of waveform features are characteristic of different possible artefacts. Some waveform features may be characteristic of more than one type of artefact. This process thus makes it efficient to detect potentially a plurality of different types of artefact in a single processing operation.

By way of one illustrative example, the processing operation could be implemented approximately as follows.

One component of the processing operation may comprise analyzing the AC signal power level of the actuator pressure signal to detect one or more waveform features in the actuator pressure signal.

A further component of the processing operation may comprise detecting negative drops in the actuator operational parameter signal.

A further component of the processing operation may comprise detecting positive spikes in the actuator operational parameter signal.

A further component of the processing operation may comprise detecting positive pressure spikes in the tissue pressure signal.

A further component of the processing operation may comprise detecting negative pressure drops in the tissue pressure signal.

A further component of the processing operation may comprise detecting ringing transients in the tissue pressure signal.

A further component of the processing operation may comprise computing the self-correlation (or autocorrelation) of the AC-component of the tissue pressure signal and detecting reductions in the self-correlation. In the event that a motion or position sensor is included as part of the hemodynamic parameter measurement apparatus (e.g. an accelerometer) to detect user motion or posture, a further component of the processing operation may comprise analyzing the motion / posture signal.

By combining and analyzing the outputs of all the individual processing operation components, it is possible to classify the type(s) of artefact present, based on knowledge of the particular combination of waveform features associated with occurrence of different artefact events.

As briefly mentioned above, optionally the method may further comprise generating a recommendation for a response action to each of, or at least a subset of, any detected artefact events.

For example, there may be generated a recommendation for further action on how to overcome or avoid repetition of the detected artefacts and thus improve the hemodynamic parameter measurements. The output of this step would be a recommendation for further action which can be communicated to the user via a user interface device, such as by means of a display and/or an acoustic output device. This could for instance be a display of a patient monitor to which the hemodynamic parameter measurement apparatus is communicatively coupled, or a dedicated display of the hemodynamic parameter measurement apparatus. The display output may for instance provide a report indicating the type of each detected artefact, and the response action recommended for each detected artefact.

In the event that an artefact event in the form of patient motion event is detected, the recommended response action may be to repeat the hemodynamic parameter measurement. This may be communicated to the clinician user. In some embodiments, additionally, feedback is provided advising the clinician user to take care to check that the patient does not move during the course of the repeated measurement.

In the event that an artefact event in the form of a user touching a fluid tube/line of the hemodynamic parameter measurement cuff is detected, the recommended response action may again be to repeat the measurement. In some embodiments, additionally, feedback is provided advising the clinician user to take care to check that the patient or clinician user does not touch the fluid-filled tube during the course of the measurement.

In the event of detection of an artefact event in the form of a stick-slip event in relation to material layers of the hemodynamic parameter measurement cuff, the recommended response action may again be to repeat the measurement. In some embodiments, the method may further comprise tracking re-occurrence of stick-slip events over a pre-defined number of measurements during a single measurement session, where a measurement session is a session of continuous application of the hemodynamic parameter measurement cuff to a single patient. If the number of stick-slip events during the measurement session exceeds a pre-defined threshold, this can be reported to the user via the user interface. A recommendation may further be communicated to the user to re-wrap and/or replace the cuff.

In the event of detection of an artefact event in the form of a cuff crack (slippage or loosening of a cuff securement means), the recommended response action may be to repeat the measurement. Furthermore, in some embodiments, the method may further comprise tracking re-occurrence of loosening or slippage (cuff cracking) events over a pre-defined number of measurements during a single measurement session (a measurement session being defined as a session of continuous application of the hemodynamic parameter measurement cuff to a single patient). If the number of cuff-cracks during the measurement session exceeds a pre-defined threshold, this may be reported to the user via the user interface, and a recommendation further communicated to the user interface recommending to re-wrap the cuff of the hemodynamic parameter measurement apparatus to the patient and repeat the hemodynamic parameter measurement, or to replace the cuff of the hemodynamic parameter measurement apparatus with a new cuff and repeat the hemodynamic parameter measurement.

In some embodiments, a recommendation to repeat a measurement may be contingent on a magnitude or severity of the artefact exceeding a pre-defined threshold. For example, in the case of cuff-crack events, an amplitude of the waveform feature corresponding to the cuff crack event may be determined and a recommended response action to repeat the measurement generated only if this amplitude exceeds a threshold. The same approach can be applied for any of the other types of artefact event mentioned in this disclosure. In some cases, different recommendations may be issued dependent on different thresholds being exceeded by the magnitude or severity of the artefact. For example, in the case of the cuff crack there may be a further, high threshold for the amplitude of the cuff-crack event, and wherein when this further higher threshold is breached, a recommended response action is communicated to the user recommending to re-fit the hemodynamic parameter measurement cuff or replace the hemodynamic parameter measurement cuff and repeat the measurement.

Alternatively or additionally, in some embodiments, a correction may be applied to the sensor signals and/or the blood pressure or other hemodynamic measurements to correct for a detected artefact.

In the case of a cuff-crack, a correction might be performed based on processing of the acquired tissue pressure signal 64. As discussed above, a cuff crack leads to a drop in the acquired actuator pressure signal (AP), with a consequent drop in the tissue pressure signal (TP). Absent any artefacts, the ideal signal waveforms take the form of a uniformly incrementing (ramping) actuator pressure baseline, with the tissue pressure baseline likewise incrementing by virtue of the increasing actuator pressure. When a cuff crack occurs, this regular ramping of the actuator pressure is disturbed, and both the AP and TP signals exhibit a downward inflection in their respective baseline. The result is effectively that the tissue pressure signal includes more than one measured signal portion for a given section of the applied (actuator) pressure ramp. This disturbs the resulting hemodynamic measurement if the tissue pressure signal is fed into the measurement algorithm without any modification. A solution is that the particular tissue pressure signal intervals obtained for the duplicated actuator pressure interval can be averaged in order to obtain a single corrected series of tissue pressure pulses over the entire range of actuator pressures in monotonically increasing order. The corrected tissue pressure signal no longer contains drops in the average or baseline tissue pressure level. The existing algorithm for the given hemodynamic measurement can be directly applied to the corrected tissue pressure signal in order to determine blood pressure and/or other hemodynamic parameters.

The averaging of the duplicated tissue pressure intervals can be achieved using a weighted average in which equal weights of 0.5 are applied to each of the duplicated tissue pressure intervals (assuming each tissue pressure signal interval is only duplicated once).

Alternatively, the weighting of the duplicated tissue pressure intervals can be a function of the signal-to-noise ratio (SNR) of each of the individual tissue pressure intervals as follows.

The SNR of each of the individual tissue pressure intervals can be determined from the amplitude frequency spectrum of the tissue pressure signal. The absolute value of the sum of the complex amplitudes of the frequency components at the pulse rate and its harmonics is indicative of the total amplitude of the cardiac signal, referred to as CardiacAmplitude_i, where integer i can be 1 or 2, corresponding to the first and second tissue pressure signal intervals as acquired during the first and second application of the repeated actuator pressure interval.

The square root of the sum of the squares of the absolute values of the amplitudes of the remaining frequency components is indicative of the total noise in the signal, referred to as NoiseAmplitude_i, where i can be 1 or 2, corresponding to the first and second tissue pressure signal intervals as acquired during the first and second application of the repeated actuator pressure interval. These measures may be used to obtain an SNR measure defined as SNR_i = CardiacAmplitude_i / NoiseAmplitude_i.

The weighting of the first and second tissue pressure signal intervals as acquired during the first and second application of the repeated actuator pressure signal interval can then be determined using the factors SNR_1 / (SNR_1 + SNR_2) and SNR_2 / (SNR_1 + SNR_2), respectively, in order to obtain a single tissue pressure signal interval over the duplicated actuator pressure interval.

Alternatively, the SNR of each of the individual tissue pressure signal intervals can be determined from the power frequency spectrum. The sum of the power of the frequency components at the pulse rate and its harmonics is indicative of the total power of the cardiac signal, referred to as CardiacPower_i, where integer i can be 1 or 2, corresponding to the first and second tissue pressure signal interval as acquired during the first and second application of the repeated actuator pressure interval. The sum of the power of the remaining frequency components is indicative of the total noise in the signal, referred to as NoisePower_i, where i can be 1 or 2, corresponding to the first and second tissue pressure signal interval as acquired during the first and second application of the repeated actuator pressure interval. These measures may be used to obtain an SNR measure defined as SNR_i = CardiacPower_i / NoisePower_i.

The weighting of the first and second tissue pressure signal intervals as acquired during the first and second application of the repeated actuator pressure signal can then be determined using the factors SNR_1 / (SNR_1 + SNR_2) and SNR_2 / (SNR_1 + SNR_2), respectively, in order to obtain a single tissue pressure signal interval over the duplicated actuator pressure interval. The algorithms of the hemodynamic measurement can be directly applied to the corrected tissue pressure signal in order to determine blood pressure and/or other hemodynamic parameters as the corrected tissue pressure signal no longer contains drops in the average tissue pressure level. In the preceding paragraphs, the averaging procedures are explained for tissue pressure intervals which have been acquired twice for a portion of the actuator pressure ramp. In case more than one cuff-crack occurs and tissue pressure intervals have been acquired three times or more for a portion of the actuator pressure ramp, the averaging procedures can be generalized to work with more than two intervals.

Furthermore, a predefined limit can be established for the maximum number of pressure intervals that may be averaged in order to obtain a correction. In case the number of duplicated pressure intervals due to cuff cracks exceeds the predefined limit, no averaging will be attempted. Instead, this may be reported to the user via the user interface, and a recommendation further communicated to the user interface recommending to re-wrap the cuff of the hemodynamic parameter measurement apparatus to the patient and repeat the hemodynamic parameter measurement, or to replace the cuff of the hemodynamic parameter measurement apparatus to the patient and repeat the hemodynamic parameter measurement.

The corrected measurement results may be displayed to the user via the user interface, optionally in addition to advice that the displayed results are corrected measurement results, so that the user can take this into account when interpreting the results and can decide whether or not a repeat of the measurement is needed. Optionally, in this case, the method may further comprise tracking how often a cuff-crack correction has been performed over the course of a predefined number of measurements within a single measurement session (a single continuous application of a cuff to a patient). If the number of corrections exceeds a predefined threshold, this may also be reported to the user and a recommendation may be generated to re-wrap and/or replace the cuff.

Furthermore, regardless of the type of artefact, in general, the process of detection of artefacts and determining the recommended response action could be implemented in at least two ways. In a first approach, the detection and classification of artefacts as well as providing the recommended follow-up action can be done after a full measurement cycle has been completed. A full measurement cycle comprises the full cycle of gradual inflation/deflation of the cuff and the acquisition of measurement signals over this time. In a second approach, the detection and classification of artefacts as well as providing the recommended response action may be performed in real-time during the course of the measurement cycle. In this case, immediately responsive to detection of an artefact event, the measurement can be directly aborted by the system. In other words, responsive to detection of an artefact, the method may comprise generating a control signal for output to the hemodynamic parameter measurement apparatus for controlling the apparatus to abort a current hemodynamic parameter measurement.

This approach saves clinical time, since continuing with a measurement affected by an artefact would lack clinical utility. Furthermore, this approach is more comfortable for the patient, since the full inflation cycle is only completed once, when good measurement results are most likely.

With regards to the recommended response actions, these can be communicated to a user via a user interface device. The user interface may include a display. The user interface may include one or more other sensory output devices, for example an acoustic output device. The recommendation and/or other advice may, by way of non-limiting example, be provided via an audio-visual message, via a visual message or via an audio message.

As briefly mentioned above, the detection of artefacts can be performed using one or more classical algorithms, or using one or more machine learning algorithms. With regards now to the use of machine learning algorithms, each of the one or more machine learning algorithms may be a classification algorithm configured to classify one or a set of signals as either containing a specific artefact or not containing the artefact. A battery of such algorithms might be included, one trained for detecting each type of artefact. Each of the one or more machine learning algorithms may be trained by making use of a database of acquired measurement data with the various types of artefacts annotated in the data. That is, the database may contain annotations of the locations of the artefacts, the duration of the artefacts, the type of artefacts and possibly the severity of the artefacts.

Although in the examples discussed above, reference has been made to use of the actuator pressure signal, more generally any operational signal indicative of an operational parameter of the pneumatic actuator can be used. Examples include for instance an actuator activity signal indicative of a pumping power level or pumping rate of a pump of the pneumatic actuator. This is in effect a proxy for the actuator pressure signal and correlated with physical disturbance of the measurement apparatus in a similar way to an actuator pressure signal. Indeed, in all of the above-described methods for detecting the particular types of artefacts, the reference to the actuator pressure signal can be replaced by a different operational parameter, such as pumping power level or pumping rate, and the method will still work in the same way.

By way of summary, the method and system of embodiments of this invention proposes an algorithm for automated artefact detection and classification that provides information about possible artefacts and thereby information about signal quality during hemodynamic parameter measurements. The proposed solution analyzes the tissue pressure signal (TP), along with a signal indicative of an operational parameter of a pneumatic actuator of the blood pressure signal, and optionally a motion sensor signal, to determine and identify the possibility of artefacts in acquired signal data. Through the detection process, a type of artefact is also determined. This can be reported through a user output, preferably also with a recommendation for further action, to the clinician / user.

The general workflow according to one set of advantageous embodiments could be summarized as follows. First, sensor signals received from the hemodynamic parameter measurement apparatus are analyzed for the presence of artefacts. This can be done based on e.g. amplitude information, correlation information, or template matching. The signals preferably include at least a tissue pressure signal indicative of a pressure between a surface of the user's body part and the cuff, and an actuator operational signal indicative of an operational parameter of the pneumatic actuator. If artefacts are detected, optionally they may be further analyzed to determine characteristics of the artefact. For example, it can be determined start and end times of the artefacts, and/or a duration of the artefacts. An amplitude of waveform features corresponding to the artefacts can be determined in order to derive a severity index for a given artefact. For example, certain artefacts discussed above are associated with an inflection in the signal. An amplitude of this inflection could be measured and used as an indicator of severity.

Once the artefacts have been located in time, they can be classified to determine whether it concerns at least one of the following types of artefacts: motion artefact, touching of the fluid tube connecting a pressure fluid sensor pad to a pressure transducer; stick-slip event in relation to layers of the cuff; and cuff-cracks (loosening or slippage of the cuff securement means).

Finally, a report can be generated for output to a user, which includes reporting of the type of artefact detected (e.g. one of the types mentioned above) and preferably a recommended follow-up action (e.g. at least one of those mentioned above).

In some embodiments, if occurrence of an artefact is detected during the course of a measurement, the method may comprise automatically and directly aborting the measurement and preferably reporting to the user the detected type of artefact and preferably providing a recommended further action. In this way, the overall measurement duration can be shortened, which saves time for the clinical team and provides greater comfort to the patient.

By detecting occurrence of artefact-causing events, embodiments of the invention provide an indicator for signal quality assessment and therefore for assessment of hemodynamic parameter measurement reliability. Embodiments therefore support clinicians in decision making by preventing errors in clinical assessment of a patient, preventing false alarms, and also in improving the clinical workflow, in particular in cases where recommendations are provided as to how to remedy repetition of artefact-causing events.

Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing unit can be implemented. The processing unit includes a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (10) for use during hemodynamic parameter measurement by a hemodynamic parameter measurement apparatus, (42) wherein the hemodynamic parameter measurement apparatus comprises a cuff for wrapping around a part of the body of a user, and wherein the cuff includes a pneumatic actuator in the form of an inflatable bladder for use in changing a pressure applied to the body part by the cuff, and further includes a tissue pressure sensor (44) arranged for sensing a pressure between a surface of the user's body part and the cuff, and an operational parameter sensing means (46) for sensing an operational parameter of the pneumatic actuator;
wherein the method (10) comprises:
receiving (12) from the hemodynamic parameter measurement apparatus a set of sensor signals, wherein the set of sensor signals includes:
a tissue pressure signal (18) indicative of said pressure between a surface of the user's body part and the cuff, and
an actuator operational signal (20) indicative of an operational parameter of the pneumatic actuator;
processing (22) both signals with an artefact detection module to detect, based on a combination of the signals, the occurrence of any of a pre-defined set of artefact events of different types, each artefact event type corresponding to a physical event affecting sensor readings; and
generating a report indicative of the type of any one or more detected artefact events.

2. The method of claim 1, wherein the artefact detection is performed based on detecting one or more characteristic features in a waveform of one or more of the received sensor signals.

3. The method of any of claims 1-2, wherein the artefact detection includes detection of a motion artefact based on: high pass filtering the actuator operational signal, and detecting a signal power level the high-pass-filtered signal which exceeds a pre-defined threshold.

4. The method of any of claims 1-3, wherein the artefact detection includes detection of a motion artefact based on computing a self-correlation measure of the tissue pressure signal, and detecting that the self-correlation measure falls below a threshold.

5. The method of any of claims 1-4, wherein the detection includes detection of a motion artefact based on:
a first detection comprising high pass filtering the actuator operational signal, and detecting a signal power level in the high-pass filtered signal at any time point which exceeds a predefined threshold; and
a second detection comprising computing a self-correlation measure of the tissue pressure signal, and detecting that the self-correlation measure at any time point falls below a threshold;
wherein a motion artefact is detected in the event that the time point to which the first detection corresponds and the time point to which the second detection corresponds are within a pre-defined proximity range of one another.

6. The method of any of claims 1-5,
wherein the set of sensor signals further includes a position and/or motion sensor signal indicative of pose and/or motion of the patient, and
wherein the method comprises detecting occurrence of an artefact event comprising a motion of the patient or change in pose of the patient.

7. The method of any of claims 1-6, wherein the artefact detection includes detection of an artefact event corresponding to a user touching a fluid line of the hemodynamic parameter measurement apparatus, and wherein the detection comprises:
computing a self-correlation measure of the tissue pressure signal over a time period; and
computing a noise measure of the actuator operational signal, the noise measure indicative of a degree of variation of the signal from a smooth ramp function over the time period;
wherein said touching is detected responsive to the self-correlation of the tissue pressure signal being below a pre-defined threshold, and the noise measure of the actuator operational signal being below a pre-defined threshold.

8. The method of any of claims 1-7 wherein the artefact detection includes detection of an artefact event corresponding to a user touching a fluid line of the hemodynamic parameter measurement cuff, and wherein the detection comprises detecting one or more pre-defined characteristic waveform patterns within the waveform of the tissue pressure signal,

9. The method of any of claims 1-8,
wherein the cuff includes a plurality of at least partially overlapping material layer portions; and
wherein the artefact detection includes detection of an artefact event corresponding to stick-slip event in relation to said layers, and wherein the stick-slip event is detected based on:
detecting an upward inflection in the tissue pressure signal exceeding a first pre-defined gradient threshold, and
detecting a downward inflection in the actuator operational signal exceeding a second pre-defined gradient threshold.

10. The method of any of claims 1-9, wherein the artefact detection includes detection of an artefact event corresponding to a loosening or slippage of a cuff securement means, and wherein the detection is based on:
detecting a downward inflection in the actuator operational signal exceeding a first pre-defined gradient threshold, and
detecting a downward inflection in the tissue pressure signal exceeding a second pre-defined gradient threshold; and
optionally wherein
the detection further comprises detecting the downward inflection in each of the tissue pressure and actuator operational signals followed by a signal portion of each respective signal representing a negative offset relative to the respective signal prior to the downward inflection; and
the detection further comprises determining whether a time duration of said signal portion exceeds a pre-defined threshold.

11. The method of any of claims 1-10,
wherein the operational parameter sensing means comprises an actuation pressure sensor for sensing a pressure within the inflatable bladder and the actuator operational signal comprises an actuator pressure signal indicative of a pressure within the inflatable bladder; and/or
wherein the actuator operational signal includes an actuator activity signal indicative of a pumping power level or pumping rate of a pump of the pneumatic actuator.

12. The method of any of claims 1-11, wherein, responsive to detection of an artefact, the method comprises generating a control signal for output to the hemodynamic parameter measurement apparatus for controlling the apparatus to abort a current hemodynamic parameter measurement.

13. A computer program product comprising code means configured for being run on a processor operatively coupled to a hemodynamic parameter measurement apparatus,
the hemodynamic parameter measurement apparatus comprising a cuff for wrapping around a part of the body of a user, and wherein the cuff includes a pneumatic actuator in the form of an inflatable bladder for use in changing a pressure applied to the body part by the cuff, and further includes a tissue pressure sensor arranged for sensing a pressure between a surface of the user's body part and the cuff, and an operational parameter sensing means for sensing an operational parameter of the pneumatic actuator; and
wherein the code means is configured, when run, to cause the processor to perform a method in accordance with any of claims 1-12.

14. A processing unit (32) adapted for a hemodynamic parameter measurement apparatus, comprising:
an input/output (34) adapted to be operatively coupled in use with a hemodynamic parameter measurement apparatus (42), the hemodynamic parameter measurement apparatus comprising a cuff for wrapping around a part of the body of a user, and wherein the cuff includes a pneumatic actuator in the form of an inflatable bladder for use in changing a pressure applied to the body part by the cuff, and further includes a tissue pressure sensor (44) arranged for sensing a pressure between a surface of the user's body part and the cuff, and further includes an operational parameter sensing means (46) for sensing an operational parameter of the pneumatic actuator;
one or more processors (36) configured to execute a method, the method comprising:
receiving from the hemodynamic parameter measurement apparatus, via the input/output, a set of sensor signals, wherein the set of sensor signals includes:
a tissue pressure signal indicative of said pressure between a surface of the user's body part and the cuff, and
an actuator operational signal indicative of an operational parameter of the pneumatic actuator;
- processing the signals with an artefact detection module to detect, based on a combination of the signals, the occurrence of any of a pre-defined set of artefact events of different types, each artefact event type corresponding to a physical event affecting sensor readings; and
generating a report indicative of the type of any one or more detected artefact events, and preferably exporting the report as a data item via the input/output.

15. A system (30), comprising
a processing unit (32) in accordance with claim 14; and
a hemodynamic parameter measurement apparatus (42), operatively coupled with the processing unit, the hemodynamic parameter measurement apparatus comprising a cuff for wrapping around a part of the body of a user, and wherein the cuff includes a pneumatic actuator in the form of an inflatable bladder for use in changing a pressure applied to the body part by the cuff, and further includes a tissue pressure sensor (44) arranged for sensing a pressure between a surface of the user's body part and the cuff, and an actuation operational parameter sensing means (46) for sensing an operational parameter of the pneumatic actuator, for example an actuator pressure sensor for sensing a pressure within the inflatable bladder.

## Patentansprüche

1. Verfahren (10) zur Verwendung während einer hämodynamischen Parametermessung mit einer hämodynamischen Parametermesseinrichtung (42), wobei die hämodynamische Parametermesseinrichtung eine Manschette zum Umwickeln eines Körperteils eines Benutzers umfasst und wobei die Manschette eine pneumatische Betätigungsvorrichtung in der Form einer aufblasbaren Blase zur Verwendung beim Ändern eines Drucks, der von der Manschette auf den Körperteil ausgeübt wird, beinhaltet und weiter einen Gewebedrucksensor (44), der zum Erfassen eines Drucks zwischen einer Oberfläche des Körperteils des Benutzers und der Manschette angeordnet ist, beinhaltet und ein Betriebsparametererfassungsmittel (46) zum Erfassen eines Betriebsparameters der pneumatischen Betätigungsvorrichtung beinhaltet;
wobei das Verfahren (10) Folgendes umfasst:
Empfangen (12) einer Menge von Sensorsignalen von der hämodynamischen Parametermesseinrichtung, wobei die Menge von Sensorsignalen Folgendes beinhaltet:
ein Gewebedrucksignal (18), das den Druck zwischen einer Oberfläche des Körperteils des Benutzers und der Manschette angibt, und
ein Betätigungsvorrichtungsbetriebssignal (20), das einen Betriebsparameter der pneumatischen Betätigungsvorrichtung angibt;
Verarbeiten (22) beider Signale mit einem Artefakterkennungsmodul zum Erkennen, basierend auf einer Kombination der Signale, des Auftretens eines beliebigen von einer vordefinierten Menge von Artefaktereignissen verschiedener Typen, wobei jeder Artefaktereignistyp einem physikalischen Ereignis entspricht, das Sensormesswerte beeinflusst; und
Erzeugen eines Berichts, der den Typ eines oder mehrerer erkannter Artefaktereignisse angibt.

2. Verfahren nach Anspruch 1, wobei die Artefakterkennung auf Basis eines Erkennens eines oder mehrerer charakteristischer Merkmale in einer Wellenform eines oder mehrerer empfangener Sensorsignale durchgeführt wird.

3. Verfahren nach einem der Ansprüche 1-2, wobei die Artefakterkennung eine Erkennung eines Bewegungsartefakts auf Basis des Folgenden beinhaltet: Hochpassfilterung des Betätigungsvorrichtungsbetriebssignals und Erkennen eines Signalleistungspegels des hochpassgefilterten Signals, das einen vordefinierten Schwellenwert überschreitet.

4. Verfahren nach einem der Ansprüche 1-3, wobei die Artefakterkennung eine Erkennung eines Bewegungsartefakts auf Basis eines Berechnens eines Selbstkorrelationsmaßes des Gewebedrucksignals und Erkennen, dass das Selbstkorrelationsmaß unter einen Schwellenwert fällt, beinhaltet.

5. Verfahren nach einem der Ansprüche 1-4, wobei die Erkennung eine Erkennung eines Bewegungsartefakts auf Basis des Folgenden beinhaltet:
eine erste Erkennung, die Hochpassfilterung des Betätigungsvorrichtungsbetriebssignals und Erkennen eines Signalleistungspegels im hochpassgefilterten Signal zu einem beliebigen Zeitpunkt, der einen vordefinierten Schwellenwert überschreitet, umfasst; und
eine zweite Erkennung, die Berechnen eines Selbstkorrelationsmaßes des Gewebedrucksignals und Erkennen, dass das Selbstkorrelationsmaß zu einem beliebigen Zeitpunkt unter einen Schwellenwert fällt, umfasst;
wobei ein Bewegungsartefakt erkannt wird, falls der Zeitpunkt, dem die erste Erkennung entspricht, und der Zeitpunkt, dem die zweite Erkennung entspricht, innerhalb eines vordefinierten Nähbereichs zueinander liegen.

6. Verfahren nach einem der Ansprüche 1-5,
wobei die Menge der Sensorsignale weiter ein Positions- und/oder Bewegungssensorsignal beinhaltet, das eine Pose und/oder Bewegung des Patienten angibt, und
wobei das Verfahren Erkennen des Auftretens eines Artefaktereignisses umfasst, das eine Bewegung des Patienten oder eine Änderung der Pose des Patienten umfasst.

7. Verfahren nach einem der Ansprüche 1-6, wobei die Artefakterkennung eine Erkennung eines Artefaktereignisses beinhaltet, das dem entspricht, dass ein Benutzer eine Flüssigkeitsleitung der hämodynamischen Parametermesseinrichtung berührt, und wobei die Erkennung Folgendes umfasst:
Berechnen eines Selbstkorrelationsmaßes des Gewebedrucksignals über einen Zeitraum; und
Berechnen eines Rauschmaßes des Betätigungsvorrichtungsbetriebssignals, wobei das Rauschmaß einen Abweichungsgrad des Signals von einer gleichmäßigen Rampenfunktion über den Zeitraum angibt;
wobei die Berührung infolgedessen erkannt wird, dass die Selbstkorrelation des Gewebedrucksignals unterhalb eines vordefinierten Schwellenwerts liegt und das Rauschmaß des Betätigungsvorrichtungsbetriebssignals unterhalb eines vordefinierten Schwellenwerts liegt.

8. Verfahren nach einem der Ansprüche 1-7, wobei die Artefakterkennung eine Erkennung eines Artefaktereignisses beinhaltet, das dem entspricht, dass ein Benutzer eine Flüssigkeitsleitung der hämodynamischen Parametermessmanschette berührt, und wobei die Erkennung Folgendes umfasst: Erkennen einer oder mehrerer vordefinierte charakteristischer Wellenformstrukturen innerhalb der Wellenform des Gewebedrucksignals.

9. Verfahren nach einem der Ansprüche 1-8,
wobei die Manschette eine Vielzahl von zumindest teilweise überlappenden Materialschichtabschnitten beinhaltet; und
wobei die Artefakterkennung eine Erkennung eines Artefaktereignisses beinhaltet, das einem Stick-Slip-Ereignis in Bezug auf die Schichten entspricht, und wobei das Stick-Slip-Ereignis auf Basis des Folgenden erkannt wird:
Erkennen eines Aufwärtsknicks im Gewebedrucksignal, der einen ersten vordefinierten Gradientenschwellenwert überschreitet, und
Erkennen eines Abwärtsknicks im Betätigungsvorrichtungsbetriebssignal, der einen zweiten vordefinierten Gradientenschwellenwert überschreitet.

10. Verfahren nach einem der Ansprüche 1-9, wobei die Artefakterkennung eine Erkennung eines Artefaktereignisses beinhaltet, das einer Lockerung oder einem Rutschen eines Manschettensicherungsmittels entspricht, und wobei die Erkennung auf Folgendes basiert:
Erkennen eines Abwärtsknicks im Betätigungsvorrichtungsbetriebssignal, der einen ersten vordefinierten Gradientenschwellenwert überschreitet, und
Erkennen eines Abwärtsknicks im Gewebedrucksignal, der einen zweiten vordefinierten Gradientenschwellenwert überschreitet; und
wobei optional
die Erkennung weiter Erkennen des Abwärtsknicks in jedem der Gewebedruck- und Betätigungsvorrichtungsbetriebssignale, gefolgt von einem Signalabschnitt jedes jeweiligen Signals, der einen negativen Offset relativ zum jeweiligen Signal vor dem Abwärtsknick darstellt, umfasst; und
die Erkennung weiter Bestimmen umfasst, ob eine Zeitdauer des Signalabschnitts einen vordefinierten Schwellenwert überschreitet.

11. Verfahren nach einem der Ansprüche 1-10,
wobei das Betriebsparametererfassungsmittel einen Betätigungsdrucksensor zum Erfassen eines Drucks innerhalb der aufblasbaren Blase umfasst und das Betätigungsvorrichtungsbetriebssignal ein Betätigungsvorrichtungsdrucksignal umfasst, das einen Druck innerhalb der aufblasbaren Blase angibt; und/oder
wobei das Betätigungsvorrichtungsbetriebssignal ein Betätigungsvorrichtungsaktivitätssignal beinhaltet, das den Pumpleistungsgrad oder die Pumprate einer Pumpe der pneumatischen Betätigungsvorrichtung angibt.

12. Verfahren nach einem der Ansprüche 1-11, wobei das Verfahren in Reaktion auf eine Erkennung eines Artefakts Erzeugen eines Steuersignals zur Ausgabe an die hämodynamische Parametermesseinrichtung umfasst, um die Einrichtung zu steuern, eine laufende hämodynamische Parametermessung abzubrechen.

13. Computerprogrammprodukt, das Codemittel umfasst, die dazu konfiguriert sind, auf einem Prozessor zu laufen, der betriebsfähig mit einer hämodynamischen Parametermesseinrichtung gekoppelt ist,
wobei die hämodynamische Parametermesseinrichtung eine Manschette zum Umwickeln eines Körperteils eines Benutzers umfasst und wobei die Manschette eine pneumatische Betätigungsvorrichtung in der Form einer aufblasbaren Blase zur Verwendung beim Ändern eines Drucks, der von der Manschette auf den Körperteil ausgeübt wird, beinhaltet und weiter einen Gewebedrucksensor, der zum Erfassen eines Drucks zwischen einer Oberfläche des Körperteils des Benutzers und der Manschette angeordnet ist, und ein Betriebsparametererfassungsmittel zum Erfassen eines Betriebsparameters der pneumatischen Betätigungsvorrichtung beinhaltet; und
wobei die Codemittel dazu konfiguriert ist, beim Laufen den Prozessor zu veranlassen, ein Verfahren gemäß einem der Ansprüche 1-12 durchzuführen.

14. Verarbeitungseinheit (32), die für eine hämodynamische Parametermesseinrichtung ausgelegt ist, umfassend:
eine Eingabe/Ausgabe (34), die dazu ausgelegt ist, in Verwendung betriebsfähig mit einer hämodynamischen Parametermesseinrichtung (42) gekoppelt zu werden, wobei hämodynamische Parametermesseinrichtung eine Manschette zum Umwickeln eines Körperteils eines Benutzers umfasst und wobei die Manschette eine pneumatische Betätigungsvorrichtung in der Form einer aufblasbaren Blase zur Verwendung beim Ändern eines Drucks, der von der Manschette auf den Körperteil ausgeübt wird, beinhaltet, und weiter einen Gewebedrucksensor (44), der zum Erfassen eines Drucks zwischen einer Oberfläche des Körperteils des Benutzers und der Manschette angeordnet ist, beinhaltet und weiter ein Betriebsparametererfassungsmittel (46) zum Erfassen eines Betriebsparameters der pneumatischen Betätigungsvorrichtung beinhaltet;
einen oder mehrere Prozessoren (36), die zum Ausführen eines Verfahrens konfiguriert sind, wobei das Verfahren umfasst:
Empfangen einer Menge von Sensorsignalen von der hämodynamischen Parametermesseinrichtung über die Eingabe/Ausgabe, wobei die Menge von Sensorsignalen Folgendes beinhaltet:
ein Gewebedrucksignal, das den Druck zwischen einer Oberfläche des Körperteils des Benutzers und der Manschette angibt, und
ein Betätigungsvorrichtungsbetriebssignal, das einen Betriebsparameter der pneumatischen Betätigungsvorrichtung angibt;
- Verarbeiten der Signale mit einem Artefakterkennungsmodul zum Erkennen, basierend auf einer Kombination der Signale, des Auftretens eines beliebigen von einer vordefinierten Menge von Artefaktereignissen verschiedener Typen, wobei jeder Artefaktereignistyp einem physikalischen Ereignis entspricht, das Sensormesswerte beeinflusst; und
Erzeugen eines Berichts, der den Typ eines beliebigen von einem oder mehrerem erkannter Artefaktereignisse angibt, und vorzugsweise Exportieren des Berichts als ein Datenelement über die Eingabe/Ausgabe.

15. System (30), umfassend
eine Verarbeitungseinheit (32) nach Anspruch 14; und
eine hämodynamische Parametermesseinrichtung (42), die betriebsfähig mit der Verarbeitungseinheit gekoppelt ist, wobei die hämodynamische Parametermesseinrichtung eine Manschette zum Umwickeln eines Körperteils eines Benutzers umfasst und wobei die Manschette eine pneumatische Betätigungsvorrichtung in der Form einer aufblasbaren Blase zur Verwendung beim Ändern eines Drucks, der von der Manschette auf den Körperteil ausgeübt wird, beinhaltet, und weiter einen Gewebedrucksensor (44), der zum Erfassen eines Drucks zwischen einer Oberfläche des Körperteils des Benutzers und der Manschette angeordnet ist, und ein Betätigungsvorrichtungsbetriebsparametererfassungsmittel (46) zum Erfassen eines Betriebsparameters der pneumatischen Betätigungsvorrichtung beinhaltet, beispielsweise einen Betätigungsvorrichtungsdrucksensor zum Erfassen eines Drucks innerhalb der aufblasbaren Blase.

## Revendications

1. Procédé (10) pour utilisation lors de la mesure de paramètres hémodynamiques par un appareil de mesure de paramètres hémodynamiques (42), dans lequel l'appareil de mesure de paramètres hémodynamiques comprend un brassard à enrouler autour d'une partie du corps d'un utilisateur, et dans lequel le brassard inclut un actionneur pneumatique sous forme de vessie gonflable permettant de modifier une pression appliquée à la partie du corps par le brassard, et inclut en outre un capteur de pression tissulaire (44) conçu pour détecter une pression entre une surface de la partie du corps de l'utilisateur et le brassard, et un moyen de détection de paramètres de fonctionnement (46) pour détecter un paramètre de fonctionnement de l'actionneur pneumatique ;
dans lequel le procédé (10) comprend :
la réception (12), en provenance de l'appareil de mesure de paramètres hémodynamiques, d'un ensemble de signaux de capteur, dans lequel l'ensemble de signaux de capteur inclut :
un signal de pression tissulaire (18) indiquant ladite pression entre une surface de la partie du corps de l'utilisateur et le brassard, et
un signal de fonctionnement d'actionneur (20) indiquant un paramètre de fonctionnement de l'actionneur pneumatique ;
le traitement (22) des deux signaux avec un module de détection d'artefacts pour détecter, sur la base d'une combinaison des signaux, l'occurrence de l'un quelconque d'un ensemble prédéfini d'événements d'artefacts de différents types, chaque type d'événement d'artefact correspondant à un événement physique affectant les lectures de capteur ; et
la génération d'un rapport indiquant le type d'un quelconque ou plusieurs événements d'artefacts détectés.

2. Procédé selon la revendication 1, dans lequel la détection d'artefacts est effectuée en fonction de la détection d'une ou plusieurs caractéristiques distinctives dans une forme d'onde d'un ou plusieurs des signaux de capteur reçus.

3. Procédé selon l'une quelconque des revendications 1-2, dans lequel la détection d'artefacts inclut la détection d'un artefact de mouvement basée sur : le filtrage passe-haut du signal de fonctionnement d'actionneur et la détection d'un niveau de puissance de signal dans le signal filtré passe-haut qui dépasse un seuil prédéfini.

4. Procédé selon l'une quelconque des revendications 1-3, dans lequel la détection d'artefacts inclut la détection d'un artefact de mouvement basée sur le calcul d'une mesure d'auto-corrélation du signal de pression tissulaire et la détection que la mesure d'auto-corrélation tombe au-dessous d'un seuil.

5. Procédé selon l'une quelconque des revendications 1-4, dans lequel la détection inclut la détection d'un artefact de mouvement basée sur :
une première détection comprenant le filtrage passe-haut du signal de fonctionnement d'actionneur, et la détection à tout moment d'un niveau de puissance de signal dans le signal filtré passe-haut qui dépasse un seuil prédéfini ; et
une seconde détection comprenant le calcul d'une mesure d'auto-corrélation du signal de pression tissulaire et la détection à tout moment que la mesure d'auto-corrélation tombe au-dessous d'un seuil ;
dans lequel un artefact de mouvement est détecté lorsque le moment correspondant à la première détection et le moment correspondant à la seconde détection se trouvent dans une plage de proximité prédéfinie l'un de l'autre.

6. Procédé selon l'une quelconque des revendications 1-5,
dans lequel l'ensemble de signaux de capteur inclut en outre un signal de capteur de position et/ou de mouvement indiquant une posture et/ou un mouvement du patient, et
dans lequel le procédé comprend la détection de l'occurrence d'un événement d'artefact comprenant un mouvement du patient ou un changement de posture du patient.

7. Procédé selon l'une quelconque des revendications 1-6, dans lequel la détection d'artefacts inclut la détection d'un événement d'artefact correspondant à un utilisateur entrant en contact avec une tubulure de fluide de l'appareil de mesure de paramètres hémodynamiques, et dans lequel la détection comprend :
le calcul d'une mesure d'auto-corrélation du signal de pression tissulaire sur une période de temps ; et
le calcul d'une mesure du bruit du signal de fonctionnement d'actionneur, la mesure du bruit indiquant un degré de variation du signal par rapport à une fonction rampe progressive sur la période de temps ;
dans lequel ledit contact est détecté en réponse à l'auto-corrélation du signal de pression tissulaire étant inférieure à un seuil prédéfini, et à la mesure du bruit du signal de fonctionnement d'actionneur étant inférieure à un seuil prédéfini.

8. Procédé selon l'une quelconque des revendications 1-7, dans lequel la détection d'artefacts inclut la détection d'un événement d'artefact correspondant à un utilisateur entrant en contact avec une tubulure de fluide du brassard de mesure de paramètres hémodynamiques, et dans lequel la détection comprend la détection d'un ou plusieurs modèles de forme d'onde caractéristiques prédéfinis dans la forme d'onde du signal de pression tissulaire,

9. Procédé selon l'une quelconque des revendications 1-8,
dans lequel le brassard inclut une pluralité de parties de couches de matériau se chevauchant au moins partiellement ; et
dans lequel la détection d'artefacts inclut la détection d'un événement d'artefact correspondant à un événement de collé-glissé en relation avec lesdites couches, et dans lequel l'événement de collé-glissé est détecté sur la base de :
la détection d'une modulation ascendante du signal de pression tissulaire dépassant un premier seuil de gradient prédéfini, et
la détection d'une modulation descendante du signal de fonctionnement d'actionneur dépassant un second seuil de gradient prédéfini.

10. Procédé selon l'une quelconque des revendications 1-9, dans lequel la détection d'artefacts inclut la détection d'un événement d'artefact correspondant à un desserrement ou à un glissement d'un moyen de fixation de brassard, et dans lequel la détection est basée sur :
la détection d'une modulation descendante du signal de fonctionnement d'actionneur dépassant un premier seuil de gradient prédéfini, et
la détection d'une modulation descendante du signal de pression tissulaire dépassant un second seuil de gradient prédéfini ; et
éventuellement, dans lequel
la détection comprend en outre la détection de la modulation descendante dans chacun des signaux de pression tissulaire et de fonctionnement d'actionneur, suivie d'une partie de signal de chaque signal respectif représentant un décalage négatif par rapport au signal respectif avant la modulation descendante ;
la détection comprend en outre la détermination pour établir si une durée de ladite partie de signal dépasse un seuil prédéfini.

11. Procédé selon l'une quelconque des revendications 1-10,
dans lequel le moyen de détection de paramètres de fonctionnement comprend un capteur de pression d'actionnement destiné à détecter une pression à l'intérieur de la vessie gonflable et le signal de fonctionnement d'actionneur comprend un signal de pression d'actionneur indiquant une pression à l'intérieur de la vessie gonflable ; et/ou
dans lequel le signal de fonctionnement d'actionneur inclut un signal d'activité d'actionneur indiquant un niveau de puissance de pompage ou un débit de pompage d'une pompe de l'actionneur pneumatique.

12. Procédé selon l'une quelconque des revendications 1-11, dans lequel, en réponse à la détection d'un artefact, le procédé comprend la génération d'un signal de commande à envoyer à l'appareil de mesure de paramètres hémodynamiques afin de commander l'appareil pour interrompre une mesure en cours de paramètres hémodynamiques.

13. Produit de programme informatique comprenant un moyen de code configuré pour être exécuté sur un processeur couplé fonctionnellement à un appareil de mesure de paramètres hémodynamiques,
l'appareil de mesure de paramètres hémodynamiques comprenant un brassard à enrouler autour d'une partie du corps d'un utilisateur, et dans lequel le brassard inclut un actionneur pneumatique sous forme de vessie gonflable permettant de modifier une pression appliquée à la partie du corps par le brassard, et inclut en outre un capteur de pression tissulaire conçu pour détecter une pression entre une surface de la partie du corps de l'utilisateur et le brassard, et un moyen de détection de paramètres de fonctionnement pour détecter un paramètre de fonctionnement de l'actionneur pneumatique ;
dans lequel le moyen de code est configuré, lorsqu'il est exécuté, pour amener le processeur à réaliser un procédé selon l'une quelconque des revendications 1-12.

14. Unité de traitement (32) adaptée à un appareil de mesure de paramètres hémodynamiques, comprenant :
une entrée/sortie (34) conçue pour être couplée fonctionnellement à un appareil de mesure de paramètres hémodynamiques (42), l'appareil de mesure de paramètres hémodynamiques comprenant un brassard à enrouler autour d'une partie du corps de l'utilisateur, et dans laquelle le brassard inclut un actionneur pneumatique sous forme de vessie gonflable permettant de modifier une pression appliquée à la partie du corps par le brassard, et inclut en outre un capteur de pression tissulaire (44) conçu pour détecter une pression entre une surface de la partie du corps de l'utilisateur et le brassard, et inclut en outre un moyen de détection de paramètre de fonctionnement (46) pour détecter un paramètre de fonctionnement de l'actionneur pneumatique ;
un ou plusieurs processeurs (36) configurés pour exécuter un procédé, le procédé comprenant :
la réception, en provenance de l'appareil de mesure de paramètres hémodynamiques, par l'intermédiaire de l'entrée/sortie, d'un ensemble de signaux de capteur, dans laquelle l'ensemble de signaux de capteur inclut :
un signal de pression tissulaire indiquant ladite pression entre une surface de la partie du corps de l'utilisateur et le brassard, et
un signal de fonctionnement d'actionneur indiquant un paramètre de fonctionnement de l'actionneur pneumatique ;
- le traitement des signaux avec un module de détection d'artefacts pour détecter, sur la base d'une combinaison des signaux, l'occurrence de l'un quelconque d'un ensemble prédéfini d'événements d'artefacts de différents types, chaque type d'événement d'artefact correspondant à un événement physique affectant les lectures de capteur ; et
la génération d'un rapport indiquant le type d'un quelconque ou plusieurs événements d'artefacts détectés, et de préférence l'exportation du rapport en tant qu'élément de données par l'intermédiaire de l'entrée/sortie.

15. Système (30) comprenant
une unité de traitement (32) selon la revendication 14 ; et
un appareil de mesure de paramètres hémodynamiques (42), couplé fonctionnellement à l'unité de traitement, l'appareil de mesure de paramètres hémodynamiques comprenant un brassard à enrouler autour d'une partie du corps d'un utilisateur, et dans lequel le brassard inclut un actionneur pneumatique sous forme de vessie gonflable permettant de modifier une pression appliquée à la partie du corps par le brassard, et inclut en outre un capteur de pression tissulaire (44) conçu pour détecter une pression entre une surface de la partie du corps de l'utilisateur et le brassard, et un moyen de détection de paramètres de fonctionnement d'actionneur (46) pour détecter un paramètre de fonctionnement de l'actionneur pneumatique, par exemple un capteur de pression d'actionneur pour détecter une pression à l'intérieur de la vessie gonflable.
